# EUROPEAN PATENT SPECIFICATION

(11) **EP 1 013 303 B1**
(45) Date of publication and mention of the grant of the patent: **27.03.2002**
(21) Application number: 99204367.9
(22) Date of filing: 17.12.1999
(51) Int. Cl.: A61N 1/05

(54) **Multi-electrode intravascular lead**
Intravaskuläre Elektrodenleitung mit mehreren Elektroden
Fil d'électrode intravasculaire à électrodes multiples

(30) Priority: 20.12.1998 IL 12764098
(43) Date of publication of application: 28.06.2000
(73) Proprietor: IMPULSE DYNAMICS N.V., Curacao (AN)
(72) Inventor: Prutchi David, TX 77566, (US)
(74) Representative: Pochart, François

(56) References cited:
- EP-A- 0 919 254
- WO-A-98/43697
- US-A- 5 755 766

## Description

### FIELD OF THE INVENTION

The present invention relates to chronically-implantable electrical leads for intravascular delivery of electrodes. More particularly it relates to a multi-electrode intravascular lead, designed for use in coronary veins and venules.

### BACKGROUND OF THE INVENTION

Chronically-implantable leads for intravascular delivery of electrodes to be positioned in the coronary veins must be of small diameter (typical dimension < 1,3 mm (4 French)), to enable effective catheterization of the smaller venules.

In addition, leads for intravascular or intracardiac chronic implants must be flexible enough to withstand the harsh cyclic loading conditions to which they are exposed. For this reason, these leads are usually constructed with one or more wire coils that impart the lead its desirable mechanical properties.

For the purpose of electrically controlling the cardiac muscle, as described by Ben-Haim et al. in WO-A-97/25098 leads must present low resistance (or low impedance in AC systems) to electric currents in order to be effective.

Resistance however is a function of material properties (bulk resistivity), cross-section of the conductor and the conductor's length. As such, the long, thin wires needed for the construction of very thin leads, and that results in high resistance values, which are highly inefficient for their use with implantable electrical muscle controllers.

For example, prior art 20 cm long, 1,0 to 1,3 mm (3 to 4 French) wide tetrapolar leads, having MP35N coated wires, forming parallel coils have terminal-to-electrode resistance values ranging from 150 Ohms to 200 Ohms. At the same time, typical electrodes for electrical muscle control have ionic conduction impedances of approximately 150 Ohms. As such, the wire resistance accounts for energy loss of as much as half the energy that could otherwise be delivered to the tissue.

Multipolar leads for permanent implant in the coronary veins are well known and are in current use for left-ventricular pacing. Daubert et al. (J.C. Daubert, P. Ritter, H. LeBreton, D. Gras, C. Leclercq, A. Lazarus, J. Mugica, P. Mabo and S. Cazeau, "Permanent left-ventricular pacing with transvenous leads inserted into the coronary veins", *PACE,* 21(1-II), 239-245, 1998) describe clinical trials that had been conducted using a specially-developed coronary-sinus lead for this purpose.

Furthermore, thin catheters for acute catheterization are commercially available. These catheters usually use parallel-cables (not coiled) to achieve a small cross-section. However parallel-cable technology for chronically-implantable leads is still under development, since leads constructed in this technology may not be as robust and durable as those made using wire coils, and therefore require careful design to achieve the required reliability. Moreover, these leads do not have an intrinsic lumen for a guidewire or stylet, and thus require the development of new positioning techniques.

The closest geometrical configuration related to the present invention of which the inventor is aware is described in US Patent No. 5,755,766 (Chastain et al.). Named "Open-ended Intravenous Cardiac Lead", that patent discloses a lead which has a narrower distal end than the rest of the lead body. However, the change in the lead diameter is caused by the reduction in the lumen diameter, bringing no electrical or structural improvements to the design of the lead. Furthermore, in that patent there is a thin lead extension, which is deployable after the lead had been positioned. However, the lead extension of the lead described therein carries electrodes through conductors which maintain their diameter along the length of the catheter and must fit within the lumen of the lead (this may prove impractical, even for high impedance connections). Again, this option neither reduces the electrical resistance of the lead, nor does it improve the overall reliability of the lead.

To-date leads are built using conductors of constant-diameter. As such, the resistivity is constant along the lead. In contrast to that, in the present invention resistivity is maintained low along most of the lead, only increasing at the distal end, where reduced diameter lead is required to effectively catheterize the coronary sinus and coronary veins.

### BRIEF DESCRIPTION OF THE INVENTION

It is the object of the present invention to provide an improved intravascular lead for the delivery of electrodes suitable for use in coronary veins.

It is another object of the present invention to provide an intravascular lead that is relatively thinner at its distal end in its diameter with respect to known intravascular leads, thus allowing insertion of the distal end of the lead and delivering of an electrode in thinner coronary venules.

Yet another object of the present invention is to provide such an intravascular lead constructed at its distal end of tapered coated cables, thus imparting lower impedance and smaller diameter intravascular lead end than prior art intravascular leads.

There is thus provided, in accordance with a preferred embodiment of the present invention, an intravascular lead comprising at least one electrode electrically connected to a conductive wire of a predetermined diameter, tapered at least at one predetermined location along said wire towards its distal end to provide a predetermined smaller diameter wire, thus presenting a main proximal relatively thick lead segment, and a relatively thinner distal lead segment, allowing catheterization of the cardiac sinus and in particular the delivery of said electrode into a selected coronary venule.

The tapering of the end of the wire renders the lead lower total impedance relatively to other prior-art leads.

### BRIEF DESCRIPTION OF THE FIGURES

In order to better understand and appreciate the present invention a detailed description of the invention is provided below, with reference to the attached Figures, in which:
- Fig. 1: illustrates a general view of a typical embodiment of the multi-electrode low-impedance intravascular lead of the present invention (a tetrapolar lead).
- Fig. 2: demonstrates the use of the same typical embodiment of the present invention in cardiac coronary veins.
- Fig. 3: depicts a see-through view of the distal end of same typical embodiment of the low-impedance intravascular lead of the present invention.
- Fig. 4: is a detailed view of longitudinal cross-section across the narrowing section of the low-impedance intravascular lead of the present invention, near its distal end.
- Fig. 5: is a longitudinal cross-section view of another embodiment of the multi-electrode intravascular lead of the present invention, having a varying diameter distal end.

### DETAILED DESCRIPTION OF THE INVENTION AND FIGURES

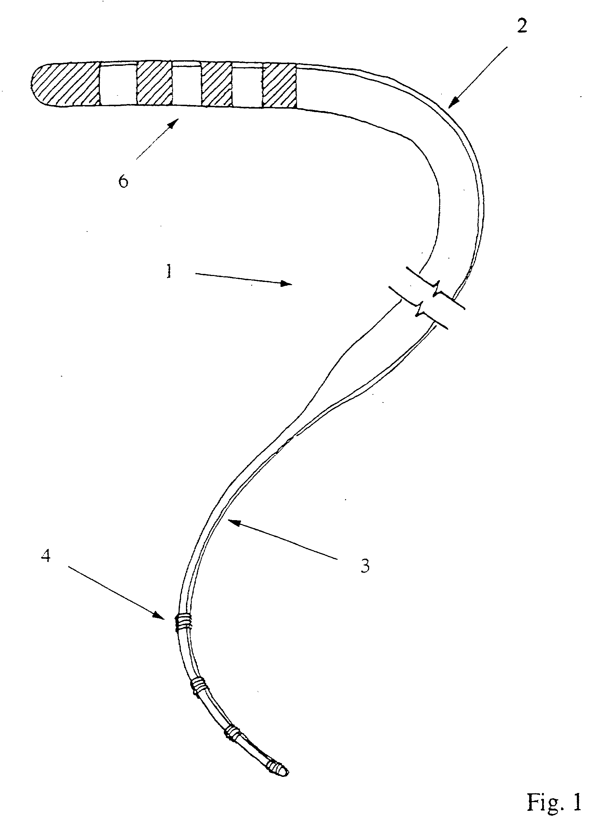

Fig. 1 illustrates a general view of a typical embodiment of the multi-electrode low-impedance intravascular lead of the present invention. The lead (assigned the general reference numeral 1) comprises a low-impedance thick lead segment (2) provided with a four-connections connector (6) at its proximal end, and a high-impedance thin lead segment (3) towards the distal end of the lead, with a four-electrode array (4). The thin segment length is predetermined by the length required in the catheterization of the cardiac sinus and cardiac venules. As shown, the lead segment (2) is thick (and thus can be made to have low resistance) along most of its length, but has a thinner segment (3) towards its distal end, allowing for more ease and convenience with the catheterization of the coronary sinus and cardiac veins.

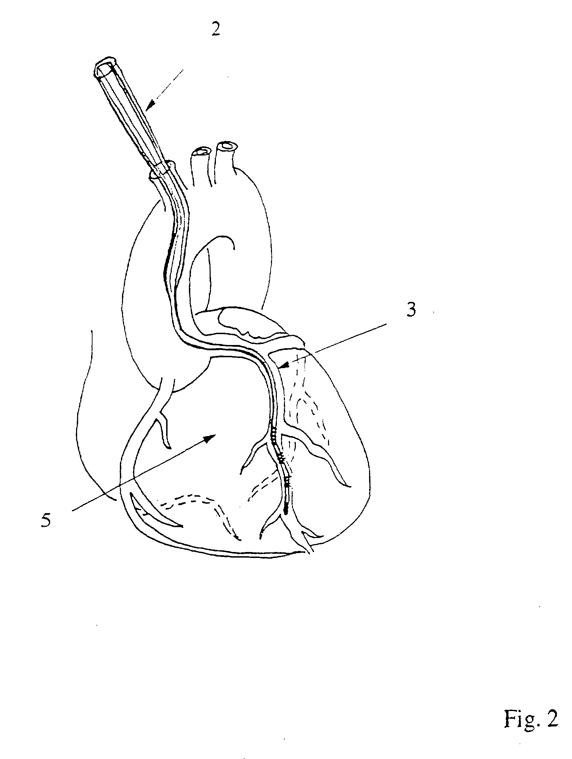

Fig. 2 illustrates the use of the same typical embodiment of the present invention in cardiac coronary veins. As shown, the thick segment (2) ,typically having a diameter of 2,0 to 2,7 mm (6 to 8 French), of the lead extends from the implantable device all the way down the vein to the right atrium (5). This thick lead segment (2) may have a length of 30 to 40 cm to enable appropriate positioning of the implantable device, as well as sufficient "slack" to accommodate displacement, rotation and growth without subsequent dislocation of the electrodes from their predetermined position. On the other hand, the thin. distal segment (3) may have a length of only 5 to 10 cm to enable catheterization of the coronary veins.

The unique configuration of the multi-electrode intravascular lead of the present invention makes it easier to be navigated through the coronary sinus and permanently located in the coronary venule of choice. At the same time, this makes it possible to have a lead with connector-to-electrode resistance of only a few tens of Ohms. For example, the thick segment can be manufactured to pose a resistance of 5 to 20 Ohms (say 15 Ohms), while the short, thin segment may add an additional resistance of 10 to 40 Ohms adding up to a total connector-to-electrode impedance of 15 to 60 Ohms (a 5 to 10-fold improvement over the prior art devices).

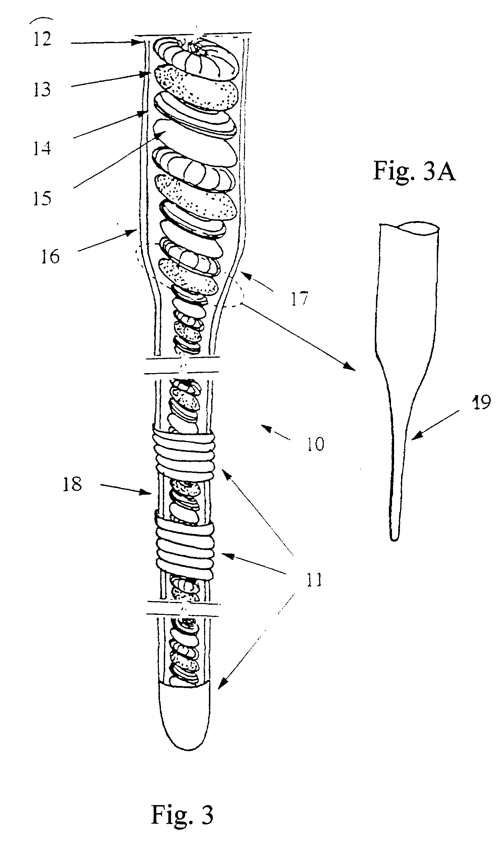

Fig. 3 shows the construction detail of a preferred embodiment of the multi-electrode low-impedance intravascular lead of the present invention.

Shown is a tetrapolar intravascular lead (generally referenced by the numeral 10), constructed in accordance with the present invention, comprising four coated insulated electrically conductive wires (12-15), coiled parallely (along a substantial portion of the lead), externally covered by an insulating jacket (16). At a transition area (17), the wires (12-15), still coiled parallely, become narrower, thus the whole lead diameter decreases, to present a thinner distal end segment (18). At the point at which it is desirable to reduce the lead diameter, the cross-section of the wires forming the parallel coils is gradually reduced. Typically, the reduction in the wire cross-section area would be in the range of 30 to 70 per cent from the original thick segment cross-section area. This is achieved by the use of tapered wires (19), one of which is depicted in Fig. 3A, shown magnified. As this happens, the outer diameter of the coils is also reduced, but at the same time, a lumen of substantially constant diameter is retained, making it possible to use the standard or tapered stylets to aid the implant and positioning of the lead. At the distal end there is an array of electrodes (11)

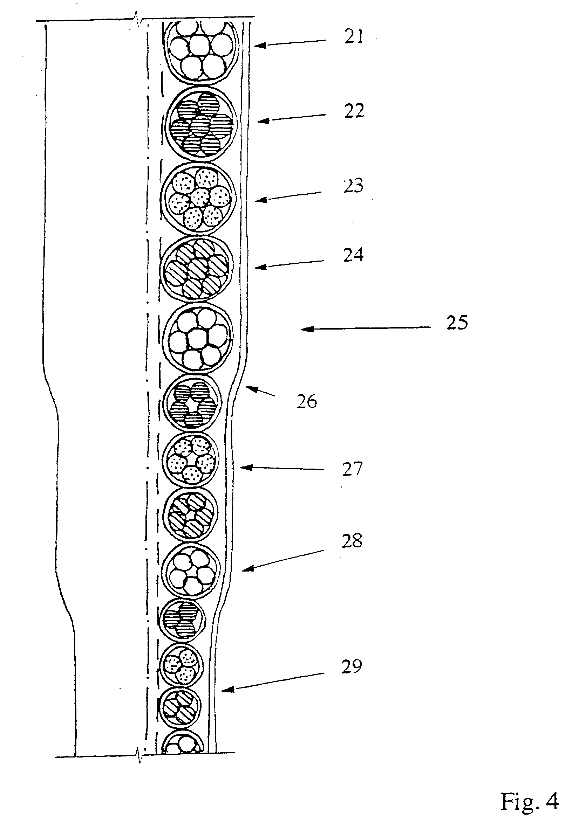

Although the use of a gradually-tapered wire is the preferred method of construction, other techniques can be equally applied in order to provide a narrowing wire. As shown in Fig. 4, if filament cables (21-24) are used as the lead constructors, then the tapering can be done discretely, by reducing the number of filaments in the wire within the transition area of the wire. At the thick segment of the lead (25), the cable is constructed of 7 filament wires. At a predetermined transitional location (26) the cables became thinner due to reduction in the number of filament wires used - in this case 5 wires (27).

Optionally, the lead can be further tapered at a second transition location (28), where the cables become narrower as the number of filament wires is reduced to 3 (29).

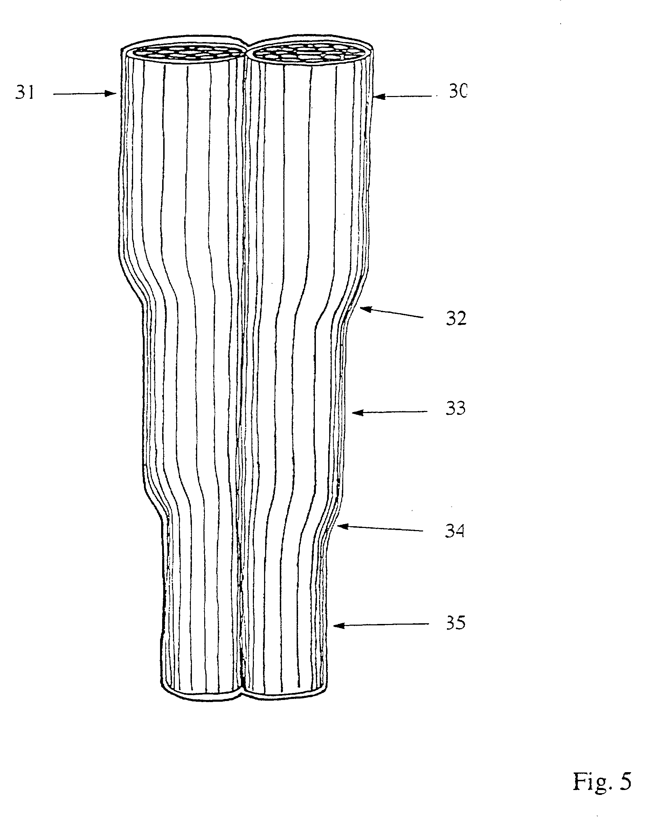

The filament cable can be constructed of coiled filament wires but also of straight parallel wires, since the same technique can be applied to form a tapered lead using parallel-cable technology. An example of how the invention applies to the development of parallel-cable leads is shown in Fig. 5., where another typical embodiment of the lead of the present invention is constructed of two parallel coated cables (30, 31). Each of the cable is tapered at a predetermined transition location (32) preferably the same for both cables, to present a narrower segment (33) - in this example the number of filament wires is reduced to achieve the narrower segment. At a second transitional location (34) is optionally provided where the lead is further tapered to yet narrower segment (35).

The advantages of the lead of the present invention are very large over the prior art leads when applied to the design of leads for Electrical Muscle Control, cardioversion, defibrilation or other applications where it is desirable to apply large electrical currents to zones of the heart by way of the coronary venules.

For example, a 30mA (initial current) pulse applied as described by Ben-Haim et al. in WO-A-97/25098 would require an initial driving voltage of approximately 18V using a prior art bipolar lead, that yields a combined lead-resistance/ionic-conductance impedance of 600 Ohms. In contrast, only 6V would be needed, using a lead constructed in a conservative manner, according to the present invention. In this same example, and considering an implantable muscle controller where the energy delivered to the tissue comes from the discharge of a tank capacitor, prior-art leads would consume 112.5% more energy than a lead constructed in accordance with the present invention.

Other applications for the disclosed lead and methods of its construction of the present invention may be found, especially those related to the delivery or acquisition of signals from tissues or organs that can be accessed only through thin catheters.

It should be clear that the description of the embodiments and attached Figures set forth in this specification serves only for a better understanding of the invention, without limiting its scope as covered by the following Claims.

It should also be clear that a person skilled in the art after reading the present specification could make adjustments or amendments to the attached Figures and above described embodiments that would still be covered by the following Claims.

## Claims

1. An intravascular lead (1) for catheterization of the cardiac sinus and in particular delivery of electrodes into a selected coronary venule, said lead having a distal end and a proximal end, comprising at least one electrode electrically connected to a conductive insulated wire (12, 13, 14, 15) of a predetermined diameter, **characterized in that** the wire is tapered towards its distal end at least at one predetermined location along its length, the lead thus presenting a main proximal relatively thick lead segment (2), and a relatively thinner distal lead segment (3).

2. The intravascular lead (1) according to claim 1, wherein more than one electrically conductive wire (12,13,14,15) is used in the construction of said lead.

3. The intravascular lead (1) according to claim 2, wherein four insulated electrically conductive wires (12-15) are used in the construction of said lead.

4. The intravascular lead (1) according to any one of claims 1-3, wherein the lead comprises a lumen of substantially constant diameter for the implanting and positioning of the lead.

5. The intravascular lead (1) according to any one of claims 1-4, wherein a four-connections electrical connector (6) is provided at its proximal end (2).

6. The intravascular lead (1) according to claim 5, wherein said distal end (3) of said lead is provided with a four-electrode array (4).

7. The intravascular lead (1) according to any one of claims 1-6, wherein said thin lead segment (3) has a predetermined length as required in the catheterisation of the coronary sinus and cardiac venules.

8. The intravascular lead (1) according to any one of claims 1-7, wherein the tapering of the wire cross-section area of the thin segment (3) of the lead would be in the range of 30 to 70 per cent from the wire cross-section area of the thick segment (2) of the lead.

9. The intravascular lead (1) according to any one of claims 1-8, wherein said main proximal relatively thick lead segment (2) diameter measures 2,0 to 2,7 mm (6 to 8 French).

10. The intravascular lead (1) according to any one of claims 1-9, wherein said proximal relatively thick lead segment (2) length measures between 30 to 40 cm.

11. The intravascular lead (1) according to any one of claims 1-10, wherein said thin distal lead segment (3) length measures between 5 to 10 cm.

12. The intravascular lead (1) according to any one of claims 1-11, wherein said proximal relatively thick lead segment (2) has a resistance of between 5 to 20 Ohms.

13. The intravascular lead (1) according to any one of claims 1-12, wherein said distal relatively thinner lead segment (3) has a resistance of between 10 to 40 Ohms.

14. The intravascular lead (1) according to any one of claims 1-13, wherein the total impedance of said lead is between 15 to 60 Ohms.

15. The intravascular lead (1) according to any one of claims 1-14, wherein said insulated electrically conductive wire (12,13,14,15) is coiled along a substantial portion of the lead.

16. The intravascular lead (1) according to any one of claims 1-15, wherein said insulated electrically conductive wire (12,13,14,15) is externally covered by an insulating jacket (16).

17. The intravascular lead (1) according to any one of claims 1-16, wherein said tapering is achieved by the use of a filament cable (21) in the construction of said lead, and wherein at a predetermined location (26,28) along said filament cable, the number of filament wires forming said cable is reduced.

18. The intravascular lead (1) according to any one of claims 1-17, wherein said lead is tapered in several locations (26,28) along said lead.

## Patentansprüche

1. Intravaskuläre Leitung (1) zum Katheterisieren des Herzsinus und insbesondere zum Leiten von Elektroden in eine ausgewählte koronare Venüle, wobei die Leitung ein distales und ein proximales Ende besitzt, umfassend mindestens eine Elektrode, die elektrisch mit einem leitenden, isolierten Draht (12, 13, 14, 15) vorbestimmten Durchmessers verbunden ist, **dadurch gekennzeichnet, daß** der Draht in Richtung seines distalen Endes zumindest an einer vorbestimmten Stelle entlang seines Längsverlaufs verjüngt ist, so daß die Leitung ein proximales, relativ dickes Haupt-Leitungssegment (2) und ein relativ dünneres distales Leitungssegment (3) aufweist.

2. Leitung (1) nach Anspruch 1, bei der mehr als ein elektrisch leitender Draht (12, 13, 14, 15) für den Aufbau der Leitung verwendet ist.

3. Leitung (1) nach Anspruch 2, bei der vier isolierte elektrisch leitende Drähte (12 - 15) bei dem Aufbau der Leitung verwendet sind.

4. Leitung (1) nach einem der Ansprüche 1 bis 3, wobei die Leitung ein Lumen im wesentlichen konstanten Durchmessers zum Implantieren und Positionieren der Leitung besitzt.

5. Leitung (1) nach einem der Ansprüche 1 bis 4, bei der ein vier Anschlüsse aufweisender elektrischer Verbinder (6) an dem proximalen Ende der Leitung vorgesehen ist.

6. Leitung (1) nach Anspruch 5, bei der das distale Ende (3) der Leitung mit einer vier Elektroden umfassenden Anordnung ausgestattet ist.

7. Leitung (1) nach einem der Ansprüche 1 bis 6, bei der das dünne Leitungssegment (3) eine vorbestimmte Länge besitzt, wie sie bei dem Katheterisieren des Koronarsinus und von Herzvenülen erforderlich ist.

8. Leitung (1) nach einem der Ansprüche 1 bis 7, bei der die Verjüngung der Draht-Querschnittsfläche des dünnen Segments (3) der Leitung in dem Bereich von 30 bis 70% derjenigen Draht-Querschnittsfläche beträgt, die das dicke Segment (2) der Leitung aufweist.

9. Leitung (1) nach einem der Ansprüche 1 bis 8, bei der das proximale, relativ dicke Haupt-Leitungssegment (2) einen Durchmesser von 2,0 bis 2,7 mm (6 bis 8 French) aufweist.

10. Leitung (1) nach einem der Ansprüche 1 bis 9, bei der das proximale, relativ dicke Leitungssegment (2) eine Längenabmessung zwischen 30 und 40 cm besitzt.

11. Leitung (1) nach einem der Ansprüche 1 bis 10, bei der das dünne, distale Leitungssegment (3) eine Längenabmessung zwischen 5 und 10 cm besitzt.

12. Leitung (1) nach einem der Ansprüche 1 bis 11, bei der das proximale, relativ dicke Leitungssegment (2) einen Widerstand zwischen 5 und 20 Ohm besitzt.

13. Leitung (1) nach einem der Ansprüche 1 bis 12, bei der das distale, relativ dünnere Leitungssegment (3) einen Widerstand zwischen 10 und 40 Ohm besitzt.

14. Leitung (1) nach einem der Ansprüche 1 bis 13, bei der die Gesamtimpedanz der Leitung zwischen 15 und 60 Ohm liegt.

15. Leitung (1) nach einem der Ansprüche 1 bis 14, bei der der isolierte, elektrisch leitende Draht (12, 13, 14, 15) über einen wesentlichen Abschnitt der Leitung hinweg gewickelt ist.

16. Leitung (1) nach einem der Ansprüche 1 bis 15, bei der der isolierte, elektrisch leitende Draht (12, 13, 14, 15) außen von einem Isoliermantel (16) überzogen ist.

17. Leitung (1) nach einem der Ansprüche 1 bis 16, bei der die Verjüngung erreicht wird durch Verwendung eines Filamentkabels (21) beim Aufbau der Leitung, wobei an einer vorbestimmten Stelle (26, 28) entlang dem Filamentkabel die Anzahl von Filamentdrähten, die das Kabel bilden, verringert ist.

18. Leitung (1) nach einem der Ansprüche 1 bis 17, bei der die Leitung an mehreren Stellen (26, 28) entlang der Leitung verjüngt ist.

## Revendications

1. Fil d'électrode intravasculaire (1) pour la cathétérisation du sinus cardiaque et en particulier la fourniture d'électrodes dans une vénule coronarienne choisie, ledit fil présentant une extrémité distale et une extrémité proximale, comprenant au moins une électrode connectée électriquement à un fil conducteur isolé (12, 13, 14, 15) d'un diamètre prédéterminé, **caractérisé en ce que** le fil conducteur a une section amoindrie en direction de son extrémité distale en au moins un emplacement prédéterminé sur sa longueur, le fil d'électrode présentant ainsi un segment proximal principal relativement épais (2), et un segment (3) distal plus étroit en comparaison.

2. Le fil d'électrode intravasculaire (1) selon la revendication 1, dans lequel plusieurs fils électriquement conducteurs (12, 13, 14, 15) sont utilisés dans la structure dudit fil d'électrode.

3. Le fil d'électrode intravasculaire (1) selon la revendication 2, dans lequel quatre fils isolés et électriquement conducteurs (12 à 15) sont utilisés dans la structure dudit fil d'électrode.

4. Le fil d'électrode intravasculaire (1) selon l'une des revendications 1 à 3, dans lequel le fil d'électrode comprend une lumière d'un diamètre sensiblement constant pour l'implantation et le positionnement du fil d'électrode.

5. Le fil d'électrode intravasculaire (1) selon l'une des revendications 1 à 4, dans lequel un connecteur (6) électrique à quatre connections est disposé au niveau de son extrémité proximale (2).

6. Le fil d'électrode intravasculaire (1) selon la revendication 6, dans lequel ladite extrémité distale (3) dudit fil d'électrode est muni d'une rangée de quatre électrodes (4).

7. Le fil d'électrode intravasculaire (1) selon l'une des revendications 1 à 6, dans lequel ledit segment distal plus étroit (3) présente une longueur prédéterminée comme requis pour la cathétérisation du sinus coronarien et des vénules cardiaques.

8. Le fil d'électrode intravasculaire (1) selon l'une des revendications 1 à 7, dans lequel le rétrécissement de la surface de section transversale du segment plus étroit (3) du fil d'électrode serait dans la plage allant de 30 à 70 pour-cents de la surface de section transversale du segment épais (2) du fil d'électrode.

9. Le fil d'électrode intravasculaire (1) selon l'une des revendications 1 à 8, dans lequel ledit diamètre dudit segment principal proximal de fil (2) relativement épais mesure 2 à 2,7 mm.

10. Le fil d'électrode intravasculaire (1) selon l'une des revendications 1 à 9, dans lequel ladite longueur dudit segment proximal de fil (2) relativement épais mesure 30 à 40 cm.

11. Le fil d'électrode intravasculaire (1) selon l'une des revendications 1 à 10, dans lequel ledit segment distal de fil plus étroit (3) mesure entre 5 et 10 cm.

12. Le fil d'électrode intravasculaire (1) selon l'une des revendications 1 à 11, dans lequel ledit segment proximal de fil relativement épais (2) présente une résistance comprise entre 5 et 20 Ohms.

13. Le fil d'électrode intravasculaire (1) selon l'une des revendications 1 à 12, dans lequel ledit segment distal de fil relativement plus étroit (3) présente une résistance comprise entre 10 et 40 Ohms.

14. Le fil d'électrode intravasculaire (1) selon l'une des revendications 1 à 13, dans lequel l'impédance totale dudit fil d'électrode est comprise entre 15 et 60 Ohms.

15. Le fil d'électrode intravasculaire (1) selon l'une des revendications 1 à 14, dans lequel ledit fil isolé électriquement conducteur (12 à 15) est enroulé sur une partie importante du fil d'électrode.

16. Le fil d'électrode intravasculaire (1) selon l'une des revendications 1 à 15, dans lequel ledit fil isolé électriquement conducteur (12 à 15) est recouvert à l'extérieur par une gaine isolante (16).

17. Le fil d'électrode intravasculaire (1) selon l'une des revendications 1 à 16, dans lequel la section amoindrie s'achève par l'utilisation d'un câble filamentaire (21) dans la structure dudit fil, et dans lequel au niveau d'un emplacement prédéterminé (26, 28) le long dudit câble filamentaire, le nombre de fils filamentaires formant ledit câble est réduit.

18. Le fil d'électrode intravasculaire (1) selon l'une des revendications 1 à 17, dans lequel ledit fil d'électrode a une section amoindrie en différents emplacements (26, 28) le long dudit fil d'électrode.
